# EUROPÄISCHE PATENTSCHRIFT

(11) **EP 2 273 987 B1**
(45) Veröffentlichungstag und Bekanntmachung des Hinweises auf die Patenterteilung: **31.07.2013**
(21) Anmeldenummer: 09745504.2
(22) Anmeldetag: 30.04.2009
(51) Int. Cl.: A61K 9/00, A61K 9/70

(54) **FILMFÖRMIGE ZUBEREITUNG MIT ÖLIGEN SUBSTANZEN ZUR ORALEN VERABREICHUNG**
FILM-SHAPED PREPARATION COMPRISING OILY SUBSTANCES FOR ORAL ADMINISTRATION
PRÉPARATION EN FORME DE FILM COMPRENANT DES SUBSTANCES HUILEUSES ET DESTINÉE À L'ADMINISTRATION ORALE

(30) Priorität: 13.05.2008 DE 102008023345
(43) Veröffentlichungstag der Anmeldung: 19.01.2011
(73) Patentinhaber: LTS LOHMANN Therapie-Systeme AG, 56626 Andernach (DE)
(72) Erfinder: MÜLLER, Markus, 53840 Troisdorf (DE); PIOTROWSKI, Holger, 76646 Bruchsal (DE)
(74) Vertreter: Schweitzer, Klaus
(86) Internationale Anmeldenummer: PCT/EP2009/003138
(87) Internationale Veröffentlichungsnummer: WO 2009/138170

(56) Entgegenhaltungen:
- WO-A-2006/078998
- DE-A1- 19 652 257
- US-A1- 2004 137 027
- US-A1- 2005 019 291
- JÜRGEN FALBE, ELISABETH HILLEN-MASKE: 'Römpp Chemie Lexikon', GEORG THIEME VERLAG, STUTTGART vol. MINERALÖLE, Seite 2799

## Beschreibung

Die vorliegende Erfindung betrifft feste, filmförmige Zubereitungen mit mindestens einer öligen Substanz zur oralen Verabreichung wie definiert in Auspruch 1.

Im Mundbereich und auf Schleimhäuten des Mundes anzuwendende flächige Darreichungsformen, sogenannte Wafer, sind bekannt. Die Patentschrift US 3,444,858 beschreibt Medikamentenstreifen auf Basis eines gelatinehaltigen Materials. Auch sind zu Anfang der 70er Jahre bereits Arzneimittel in Folienform beschrieben worden, beispielsweise im New England Journal of Medicine, 289, 533-535 (1973). Die Gebrauchsmusterschrift DE 24 49 865 beschreibt Arzneistoffträger in Folienform, die unterschiedliche Wirkstoffe und Wirkstoffkonzentrationen enthalten.

Die US-Patentschrift Nr. 4,128,445 offenbart technische Lösungen bei der Beladung von Trägermaterial mit Wirkstoffen und geht dabei auf die nachträgliche Zugabe von Wirkstoffzubereitungen auf vorgefertigte folienförmige Zubereitungen ein. Es werden Beladungsverfahren in trockener und feuchter Form beschrieben, die eine gleichmäßige, nachträgliche Verteilung von Wirkstoff auf einer Schicht zum Ziel haben.

Bekannt sind auch Vorschläge für wirkstoffbeladene Folien außerhalb einer Anwendung im medizinischen Bereich. So wird in der EP 0 219 762 eine wasserlösliche Folie aus Stärke, Gelatine, Glycerin oder Sorbit offenbart, die mittels Walzenauftragsverfahren beschichtet ist. Dabei wird erwähnt, daß sich solche Dosierungsformen auch mit Aromastoffen herstellen lassen.

Die US2004/137027 A1 offenbart Film-Zubereitungen zur oralen Verabreichung enthaltend 0,5 bis 30% Aromastoffe wie ätherische Öle in einer Matrix aus mindestens 40% Polyvinyl Alkohol.

Eine zur Herstellung folienförmiger aromastoffhaltiger Zubereitungen geeignete Formulierung wird in der EP 0 460 588 beschrieben. Besondere Vorteile werden in der Zusammensetzung aus 20 bis 60 Gew.-% Filmbildner, 2 bis 40 Gew.-% Gelbildner, 0,1 bis 35 Gew.-% Wirk- oder Aromastoff und maximal 40 Gew.-% eines inerten Füllstoffs gesehen. Als Gelbildner ist neben anderen Verbindungen Polyvinylalkohol genannt. Es erweist sich jedoch, daß die gelbildenden Eigenschaften von Polyvinylalkohol mit den in dieser Druckschrift genannten Filmbildnern nur bedingt verträglich sind. Ein Anteil von 20 und mehr Gewichtsanteilen Filmbildner - zumeist ein Zuckerderivat, Polyethylenglycol oder dergleichen - führte bereits bei der herstellungsbedingten Trocknung in dünner Schicht zu einem erheblichen Aromaverlust.

Aromastoffhaltige flächenförmige Darreichungsformen zur Anwendung im Mundbereich sind auch aus der EP 0 452 446 bekannt, jedoch werden keine Maßnahmen beschrieben, wie eine Aromastoffverdampfung bei der Herstellung und/oder Lagerung verhindert werden kann. Als Lösung dieses Problems offenbart DE 196 52 257 einzeln dosierte, folienförmige Darreichungsformen, die bei Kontakt mit Flüssigkeit schnell zerfallen und sich unter Vermeidung von Wirkstoffverlusten bei ihrer Herstellung und Lagerung herstellen lassen. Diese folienförmigen Darreichungsformen weisen eine innere, fettlösliche Phase in Form von Tröpfchen auf, in denen sich der Aromastoff befindet und die in einer äußeren, festen, aber wasserlöslichen Phase verteilt vorliegen, wobei die äußere Phase, bezogen auf die wasserfreien Anteile, mindestens 40 Gew.-% Polyvinylalkohol, bis zu 30 Gew.-% einer oberflächenaktiven Substanz und 0,1 bis 30 Gew.-% an innerer Phase, bezogen auf die äußere Phase, umfaßt.

Filmförmige Zubereitungen für eine Anwendung im Mund sind als wirkstoffhaltige oder aromastoffhaltige Darreichungsformen bereits im Handel erhältlich. Beispielsweise werden hauchdünne Streifen, die ohne Lutschen oder Kauen ein kühles, atemfrisches Geschmackserlebnis auf der Zunge hinterlassen, in den Geschmacksrichtungen "Peppermint", "Wild-Mint" und "Lemon-Frost" von der Firma Wrigley unter dem Namen ECLIPSE FLASH™ oder von der Firma Pfizer in den Geschmacksrichtungen "Cool Mint^{®}", "FreshBurst^{®}", Cinnamon und "Fresh Citrus" unter dem Markennamen Listerine PocketPaks^{®} angeboten.

Für den raschen Zerfall oder das rasche Auflösungsvermögen von filmförmigen Zubereitungen bei Kontakt mit Speichel werden hydrophile, filmbildende Polymere verwendet.

Sofern jedoch ölige Substanzen mit einer sich im Mundraum schnell auflösenden, filmförmigen Zubereitung verabreicht werden sollen, führen die unterschiedlichen Eigenschaften der Matrix (= hydrophiles filmbildendes Polymer) und der in die Matrix einzuarbeitenden öligen Substanz (hydrophob) zu Problemen.

Da hydrophile Polymere üblicherweise nur eine geringe Aufnahmekapazität für lipophile Substanzen aufweisen, ist die Menge an öliger Substanz begrenzt, mit der hydrophile Filme beladen werden können. Diese Begrenzung wird besonders deutlich, wenn größere Mengen einer öligen Flüssigkeit mit einem hydrophilen Polymer zu einem Film verarbeitet werden, denn dabei kommt es zu einem Ausschwitzen der öligen Substanz, zum Beispiel durch Phasenseparation bei der Masseherstellung (Auftrennung der O/W-Emulsion) oder später bei der Lagerung der Filme.

Für bestimmte Anwendungen ist es jedoch erforderlich, einen großen Anteil an öliger Substanz in einer filmförmigen Zubereitung unterzubringen. Beispielsweise kann es zur Erzielung gewünschter therapeutischer Wirkungen notwendig sein, den Film mit einer großen Menge eines öligen Wirkstoffs zu beladen. Zum Beispiel wird für den Wirkstoff Simethicon, der als Carminativum eingesetzt wird, eine Dosis von etwa 80 mg als therapeutisch wirksam angezeigt. Diese Menge wird zum Beispiel mit Hilfe bekannter flüssiger Darreichungsform verabreicht. Für eine Verabreichung mit Hilfe einer im Mund schnell zerfallenden, folienförmigen Zubereitung wäre diese Menge jedoch zu groß, um stabile Filme herstellen zu können.

Um die Stabilitätsprobleme zu umgehen, die bei Beladung einer hydrophilen Folie mit einer hydrophoben Substanz auftreten, werden einer Masse aus hydrophilem Polymer und hydrophober, flüssiger beziehungsweise öliger Substanz üblicherweise grenzflächenaktive Substanzen zugesetzt, d. h. Tenside oder Emulgatoren, die die Grenzflächenspannung zwischen den Phasen reduzieren. Beispielsweise offenbart US-Patent Nr. 6,177,096 die Herstellung von filmförmigen Darreichungsformen unter Zusatz von grenzflächenaktiven Substanzen.

Allerdings ist die Verwendung von grenzflächenaktiven Substanzen bei oralen Zubereitungen oftmals nicht erwünscht, denn ihre Verwendung kann negative Auswirkungen auf den Geschmackseindruck haben, indem sie zu einem seifigen Beigeschmack führen können. Zudem schränkt die Menge der zum filmbildenden Polymer zugesetzten grenzflächenaktiven Substanzen die mögliche Gesamtbeladung der Folie mit öliger Substanz ein, sofern die grenzflächenaktiven Substanzen selber nicht ebenfalls filmbildende Eigenschaften haben.

Die der vorliegenden Erfindung zugrundeliegende Aufgabe bestand daher darin, filmförmige, bei Kontakt mit Speichel oder anderen wäßrigen Flüssigkeiten schnell zerfallende Zubereitungen bereitzustellen, die eine große Menge mindestens einer öligen Substanz stabil enthalten können, ohne zusätzliche grenzflächenaktive Substanz(en) aufzuweisen.

Überraschenderweise wurde gefunden, daß bestimmte filmbildende Polymere auch ohne den Zusatz weiterer grenzflächenaktiver Substanzen in der Lage sind, größere Mengen an öligen Substanzen zu stabilisieren, und daß sich daraus stabile Filme herstellen lassen.

Bei den filmbildenden Polymeren, die auch ohne Zusatz weiterer grenzflächenaktiver Substanzen in der Lage sind, größere Mengen mindestens einer öligen Substanz zu stabilisieren, handelt es sich um vollhydrolysierte Polyvinylalkohole und teilhydrolysierte Polyvinylalkohole.

Polyvinylalkohole sind wasserlösliche Polymere des Vinylalkohols. Vinylalkohol liegt in der Regel in der tautomeren Form des Acetaldehyds vor. Daher kann Polyvinylalkohol nicht durch Polymerisation seiner Monomere hergestellt werden, sondern wird durch Verseifung von Polyvinylacetat gewonnen. Dazu wird Polyvinylacetat mit Natriumhydroxid verseift beziehungsweise hydrolysiert. Die Polyvinylalkohole, die nicht vollständig mit Natriumhydroxid verseift wurden, enthalten Vinylalkohol- und Vinylacetat-Einheiten. Diese Polyvinylalkohole werden als teilhydrolysierte Polyvinylalkohole bezeichnet.

Die Verstärkung der oberflächenaktiven Wirkung anderer Tenside durch Polyvinylalkohol ist bekannt. Ebenso das gute Emulgiervermögen (Kosmetik, Hautfilm, US 2005/0019291 A1). Die gleichzeitige Verwendung von Polyvinylalkohol als Filmbildner und (unter Umständen einziger) Emulgator zur Herstellung stabiler fester Filme mit einer öligen Komponente zur oralen Applikation wird zwar in DE 196 52 257 A1 genannt, jedoch ist nach den Angaben in dieser Druckschrift der Anteil der inneren (öligen) Phase auf maximal 30% (g/g) beschränkt und der Anteil des Filmbildners beträgt mindestens 40% (g/g), jeweils bezogen auf das wasserfreie Gesamtsystem.

Die erfindungsgemäßen wasserlöslichen, festen, filmförmigen Zubereitungen umfassen dagegen 30 bis 80 Gew.-%, vorzugsweise 40 bis 60 Gew.-% mindestens einer öligen Substanz und 5 bis 70 Gew.-%, in der bevorzugten Ausführungsform 5 bis 60 Gew.-%, mindestens eines teilhydrolysierten oder vollhydrolysierten Polyvinylalkohols. In einer bevorzugten Ausführungsform umfaßt die wasserlösliche, filmförmige Zubereitung teilhydrolysierte Polyvinylalkohole, insbesondere teilhydrolysierte Polyvinylalkohole mit einem Hydrolysegrad von etwa 88 %, das bedeutet mit einem Hydrolysegrad von 86,7 bis 88,7 %.

In einer besonders bevorzugten Ausführungsform weisen die teilhydrolysierten Polyvinylalkohole einen Hydrolysegrad von etwa 88 %, und eine Viskosität zwischen 4 und 40 mPa·s als 4 %-ige wäßrige Lösung bei 20 °C auf. Als Beispiele für besonders bevorzugte teilhydrolysierte Polyvinylalkohole können die unter dem Handelsnamen Mowiol^{®} von der Firma Kuraray Specialities Europe GmbH erhältlichen Polyvinylalkohole Mowiol^{®} 5-88, Mowiol^{®} 8-88, Mowiol^{®} 13-88, Mowiol^{®} 18-88, Mowiol^{®} 23-88, Mowiol^{®} 26-88 und Mowiol^{®} 32-88 und vergleichbare Produkte genannt werden.

Als ölige Substanzen werden bei Raumtemperatur (20°C bis 23°C) und flüssige, in Wasser schwer oder nicht lösliche Substanzen, Mischungen solcher Substanzen und solche Substanzen mit darin gelösten Bestandteilen verstanden. Zu den öligen Substanzen gemäß Anspruch 1 zählen Verbindungen wie Mineralöle (Kohlenwasserstoffe, die überwiegend durch Destillation und Raffination aus Erdöl gewonnen wurden), Syntheseöle, synthetisch hergestellte Öle, die Ester von Dicarbonsäuren sind und Siliconöle. Wahlweise können zusätzlich mindestens eine ölige Substanz aus der Gruppe der fetten Öle und ätherischen Öle (zum Großteil aus Terpenen bestehende Extrakte aus Pflanzen und Pflanzenteilen, sowie deren synthetische Kopien) vorhanden sein.

Bei den öligen Substanzen kann es sich um pharmazeutische Wirkstoffe wie Dimethicon oder Simethicon handeln, die öligen Substanzen können aber auch andere Funktionen, zum Beispiel als Aromastoff, haben.

Bei bevorzugten Ausführungsformen ist die ölige Substanz Dimethicon oder Simethicon. Dimethicon, chemisch α(Trimethylsilyl)-ω-methylpoly[oxy(dimethylsilylen)], ist eine klare, farblose Flüssigkeit, die mit Chloroform oder Ether gemischt werden kann, jedoch weder mit Wasser noch mit Ethanol mischbar ist. Bei Simethicon handelt es sich um mit Siliciumdioxid vermischtes Dimethicon. Bei beiden Substanzen handelt es sich um Arzneistoffe, die als Schaumverhütungsmittel peroral verabreicht werden und Völlegefühl und Schmerzen lindern können, die durch zu viel Gas im Magen-Darm-Trakt ausgelöst werden. Simethicon ist als Kautablette oder in flüssiger Form als Arzneimittel erhältlich. Präparatenamen für die im Handel erhältlichen Arzneimittel sind beispielsweise Elugan^{®}, Endo-Paractol^{®}, Espumisan^{®}, Imogas^{®}, Lefax^{®} und Simethicon-ratiopharm^{®}.

Weitere ölige Substanzen, die in der Zubereitung enthalten sein können, sind Menthol, Bitterfenchelöl, Pfefferminzöl, Kümmelöl.

Pfefferminzöl (Menthae piperitae aetheroleum) ist ein ätherisches Öl, das aus der Pfefferminze (Mentha *piperita*) gewonnen wird. Pfefferminzöl wird als Carminativum verwendet und wegen seiner verdauungsfördernden und entblähenden Eigenschaften zur Behandlung von Spasmen im oberen Gastrointestinaltrakt sowie der Gallenwege verabreicht.

Hauptbestandteil des Pfefferminzöls sind Menthol und Menthon. Menthol ist ein monozyklischer Monoterpen-Alkohol, der mit 0,4 g/l schlecht in Wasser löslich ist. Bei Raumtemperatur ist Menthol ein farbloser, kristalliner Feststoff.

Kümmelöl (Carvi aetheroleum) ist ein ätherisches Öl, das aus den vollreifen Früchten der Kümmelpflanze (*Carum carvi* L.) gewonnen wird. Es wird auch als Carminativum eingesetzt.

Bitterfenchelöl ist ein ätherisches Öl aus dem deutschen Fenchel (Foeniculi amari fructus), das durch Wasserdampfdestillation aus den Samen gewonnen wird. Bitterfenchelöl wirkt unter anderem verdauungsfördernd und krampflösend im Magen-Darmbereich.

Die erfindungsgemäße Zubereitung kann aus 5 bis 70 Gew.-% mindestens eines teil- oder vollhydrolysierten Polyvinylalkohols 30 bis 80 Gew.-% mindestens einer öligen Substanz bestehen. vorzugsweise beträgt der Anteil an teil- und/oder vollhydrolysiertem Polyvinylalkohol 40 bis 60 Gew.-% und der Anteil an öliger Substanz 40 bis 60 Gew.-%.

In einer bevorzugten Ausführungsform besteht die erfindungsgemäße filmförmige Zubereitung, bezogen auf den Trockenanteil, aus 40 Gew.-% mindestens eines teil- oder vollhydrolysierten Polyvinylalkohols und 60 Gew.-% mindestens einer öligen Substanz, vorzugsweise Dimethicon oder Simethicon.

In besonders bevorzugten Ausführungsformen enthält die Zubereitung neben Dimethicon oder Simethicon mindestens ein ätherisches Öl aus der Gruppe, die Pfefferminzöl, Bitterfenchelöl und Kümmelöl umfaßt.

Die Erfindung beschränkt sich jedoch nicht auf wasserlösliche, feste, filmförmige Zubereitungen aus mindestens einem Polyvinylalkohol und einer oder mehrerer öligen Substanzen. In bevorzugten Ausführungsformen kann die filmförmige Zubereitung weitere hydrophile Polymere enthalten, auch wenn mit diesen zusätzlichen Polymeren keine stabilen Filme mit einer öligen Substanz in großer Menge erzeugt werden können, sofern nur diese Polymere als Matrixmaterial verwendet werden und keine weiteren grenzflächenaktiven Substanzen zugegeben werden.

Bevorzugt sind die zusätzlichen hydrophilen Polymere aus der Cellulose und Cellulosederivate, Polyvinylpyrrolidone, Polyethylenoxide, Pullulan, hydroxypropylierte Tapiokastärke und Alginate umfassenden Gruppe ausgewählt. In einer besonders bevorzugten Ausführungsform sind die zusätzlichen hydrophilen Polymere aus der Gruppe ausgewählt, die aus Hydroxypropylmethylcellulose und Natriumcarboxymehtylcellulose besteht.

Die filmförmigen Zubereitungen können weitere Hilfs- oder Zusatzstoffe enthalten. Beispielsweise können der Polymermasse zur Herstellung der filmförmigen Zubereitung Glycerin, Sorbidex, Sucralose, Menthol und/oder Farbstoffe zugesetzt werden.

Die Anteile, in denen die bevorzugten Komponenten in der filmförmigen Zubereitung enthalten sein können, sind in Tabelle 1 zusammengefaßt:

**Tabelle 1: Mengenangaben der in der Zubereitung enthaltenen Komponenten**

| Komponente | Anteil |
|---|---|
| Polyvinylalkohol (voll-/teilhydrolysiert) | 5-70 Gew.-% |
| Ölige Komponente | 30-80 Gew.-% |
| Hydroxypropylmethylcellulose | 0-35 Gew.-% |
| Natriumcarbocymethylcellulose | 0-35 Gew.-% |
| Glycerin | 0-20 Gew.-% |
| Sorbidex | 0-20 Gew.-% |
| Sucralose | 0- 2 Gew.-% |
| Menthol | 0-10 Gew.-% |
| Bitterfenchelöl | 0- 5 Gew.-% |
| Pfefferminzöl | 0- 5 Gew.-% |
| Kümmelöl | 0- 5 Gew.-% |
| Summe | 100 Gew.-% |

Die Erfindung erstreckt sich auch auf Verfahren zur Herstellung der erfindungsgemäßen Zubereitungen. Die erfindungsgemäßen Zubereitungen können hergestellt werden, indem eine Masse, die mindestens ein filmbildendes Polymer aus der Gruppe der voll- und teilhydrolysierten Polyvinylalkohole und mindestens 30 Gew.-%, bezogen auf den Trockenanteil der Zubereitung, einer wasserunlöslichen, öligen Flüssigkeit in einem wäßrigen Lösungsmittel, vorzugsweise Wasser, hergestellt wird. Mit dieser Masse wird dann eine Unterlage beschichtet und die resultierende Beschichtung getrocknet.

Die filmförmigen Zubereitungen, die als ölige Substanz Dimethicon, Simethicon, Pfefferminzöl, Bitterfenchelöl und/oder Kümmelöl enthalten, können als Carminativum verwendet werden, denn mit ihnen können Beschwerden im Bereich des Magen-Darm-Traktes, wie Völlegefühl, Krämpfe und/oder Blähungen behandelt und gelindert werden.

### Beispiele

Um die Aufnahmekapazität hydrophiler, filmbildender Polymere für ölige Substanzen genauer zu untersuchen, wurden unterschiedliche Mengen Simethicon in Kombination mit unterschiedlichen Polymeren zu Filmen verarbeitet, indem zunächst eine wäßrige Polymerlösung mit Simethicon vermischt wurde. Mit dieser Mischung wurde eine Unterlage beschichtet und die Beschichtung wurde getrocknet.

### Beispiel 1

Es wurde eine homogene Masse der folgenden Zusammensetzung hergestellt:

| | |
|---|---|
| 16 Gew.-% | Polyvinylalkohol (Mowiol^{®} 8-88, teilhydrolysierter Polyvinylalkohol, Hydrolysegrad: 85-89 %, Viskosität (4% in H₂O, 20°C): 7 - 9 mPa·s) |
| 24 Gew.-% | Simethicon |
| 60 Gew.-% | Wasser |

Mit dieser Masse hergestellte Filme blieben stabil.

### Beispiel 2

Es wurde eine homogene Masse der folgenden Zusammensetzung hergestellt:

| | |
|---|---|
| 14 Gew.-% | Natriumcarboxymethylcellulose (Walocel^{®} CRT 30 GA; DS: 0,65-1,45, Viskosität (2%): 20 - 40 mPa·s) |
| 6 Gew.-% | Simethicon |
| 80 Gew.-% | Wasser |

Mit dieser Masse ließen sich jedoch aufgrund von Benetzungsproblemen keine Beschichtungen ohne Fehlstellen erzeugen.

### Beispiel 3:

Es wurde eine homogene Masse der folgenden Zusammensetzung hergestellt:

| | |
|---|---|
| 21 Gew.-% | Polyvinylpyrrolidon (Kollidon^{®} 90; K-Wert: 81,0 - 96,3)) |
| 9 Gew.-% | Simethicon |
| 70 Gew.-% | Wasser |

Aus dieser Masse hergestellte Filme schwitzten Simethicon aus.

### Beispiel 4:

Es wurde eine homogene Masse der folgenden Zusammensetzung hergestellt:

| | |
|---|---|
| 17,5 Gew.-% | Polyethylenoxid (Polyox^{®} WSR N80; Viskosität (5%, 25°C): 55 - 90 cP) |
| 7,5 Gew.-% | Simethicon |
| 75,0 Gew.-% | Wasser |

Aus dieser Masse hergestellte Filme schwitzten Simethicon aus.

### Beispiel 5:

Es wurde eine homogene Masse der folgenden Zusammensetzung hergestellt:

| | |
|---|---|
| 28 Gew.-% | Pullulan (PI-20; Viskosität: 128 mm²/s) |
| 12 Gew.-% | Simethicon |
| 60 Gew.-% | Wasser |

Mit dieser Masse ließen sich aufgrund von Benetzungsproblemen keine Beschichtungen ohne Fehlstellen erzeugen. Zudem schwitzten die aus dieser Masse hergestellten Filme Simethicon aus.

### Beispiel 6:

Es wurde eine homogene Masse der folgenden Zusammensetzung hergestellt:

| | |
|---|---|
| 4,4 Gew.-% | Natriumalginat (Manucol^{®} LDP MCLLDP 25BG; Viskosität (1%): 4,00-15,00) |
| 6,6 Gew.-% | Simethicon |
| 89,0 Gew.-% | Wasser |

Aus dieser Masse hergestellte Filme schwitzten Simethicon aus.

### Beispiel 7:

Es wurde eine homogene Masse der folgenden Zusammensetzung hergestellt:

| | |
|---|---|
| 10,4 Gew.-% | Hydroxypropylcellulose (Klucel^{®} LF; Viskosität (5%): 75-150 mPa·s) |
| 15,6 Gew.-% | Simethicon |
| 74,0 Gew.-% | Wasser |

Aus dieser Masse hergestellte Filme schwitzten Simethicon aus.

Wie Beispiel 1 verdeutlicht, ließen sich aus teilhydrolysiertem Polyvinylalkohol stabile Filme herstellen, die einen Anteil von 60 Gew.-% Simethicon, bezogen auf den Trockenanteil, enthielten. Mit anderen hydrophilen, filmbildenden Polymeren konnten keine stabilen Filme hergestellt werden, wie die als Vergleichsbeispiele anzusehenden Beispiele 2 bis 8 zeigen.

### Beispiel 8:

Beispielrezeptur für einen stabilen Film mit Simethicon:

| | |
|---|---|
| 27,48 Gew.-% | Mowiol 8-88 |
| 58,00 Gew.-% | Simethicon |
| 10,00 Gew.-% | Pfefferminzaroma |
| 1,00 Gew.-% | Sucralose |
| 2,50 Gew.-% | Sorbidex |
| 1,00 Gew.-% | Titandioxid |
| 0,02 Gew.-% | Patentblau |

### Beispiel 9:

Beispielrezeptur für einen stabilen Film mit Simethicon:

| | |
|---|---|
| 26,09 Gew.-% | Mowiol 8-88 |
| 7,00 Gew.-% | Pharmacoat 606 (Hydroxypropylmethylcellulose; Substitutionstyp 2910, Viskosiät (2 Gew.-% in H₂O): 4,8 - 7,2 mPa·s) |
| 0,50 Gew.-% | Walocel CRT 30 GA |
| 57,14 Gew.-% | Simethicon |
| 3,00 Gew.-% | Glycerin |
| 3,00 Gew.-% | Sorbidex |
| 0,35 Gew.-% | Sucralose |
| 0,40 Gew.-% | Menthol |
| 1,00 Gew.-% | Bitterfenchelöl |
| 1,00 Gew.-% | Pfefferminzöl |
| 0,50 Gew.-% | Kümmelöl |
| 0,02 Gew.-% | Yellow No. 6 |

## Patentansprüche

1. Wasserlösliche, feste, filmförmige Zubereitung zu oralen Verabreichung, umfassend mindestens ein filmbildendes Polymer aus der Gruppe der voll- und teilhydrolysierten Polyvinylalkohole und mindestens eine ölige Substanz, die in das filmbildende Polymer eingearbeitet ist und deren Anteil mindestens 30 Gew.-%, bezogen auf den Trockenanteil der Zubereitung, beträgt, wobei die Zubereitung mindestens eine aus der aus Mineralölen und synthetischen Ölen, welche Ester von Dicarbonsäuren sind, und Siliconölen bestehenden Gruppe ausgewählte ölige Substanz enthält, sowie wahlweise zusätzlich mindestens eine aus der aus fetten Ölen und ätherischen Ölen bestehenden Gruppe ausgewählte ölige Substanz.

2. Zubereitung nach Anspruch 1, **dadurch gekennzeichnet, daß** der teilhydrolysierte Polyvinylalkohol aus der Gruppe der teilhydrolysierten Polyvinylalkohole ausgewählt ist, die einen Hydrolysegrad von etwa 88 % aufweisen.

3. Zubereitung nach Anspruch 1 oder 2, **dadurch gekennzeichnet, daß** der teilhydrolysierte Polyvinylalkohol bei 20 °C eine Viskosität von 4 bis 40 mPa·s als 4 %-ige wäßrige Lösung aufweist.

4. Zubereitung nach Anspruch 1, **dadurch gekennzeichnet, daß** die ölige Substanz aus der Gruppe ausgewählt ist, die aus Simethicon und Dimethicon besteht.

5. Zubereitung nach einem der vorangehenden Ansprüche, **dadurch gekennzeichnet, daß** der Anteil der öligen Substanz mindestens 40 Gew.-%, vorzugsweise mindestens 50 Gew.-%, bezogen auf den Trockenanteil der Zubereitung, beträgt.

6. Zubereitung nach einem der vorangehenden Ansprüche, **dadurch gekennzeichnet, daß** der Anteil an öliger Substanz höchstens 80 Gew.-%, vorzugsweise höchstens 60 Gew.-%, bezogen auf den Trockenanteil der Zubereitung, beträgt.

7. Zubereitung nach einem der vorangehenden Ansprüche, **dadurch gekennzeichnet, daß** sie mindestens eine ölige Substanz aus der aus Bitterfenchelöl, Pfefferminzöl und Kümmelöl bestehenden Gruppe enthält.

8. Zubereitung nach einem der vorangehenden Ansprüche, **dadurch gekennzeichnet, daß** sie mindestens ein weiteres hydrophiles Polymer umfaßt.

9. Zubereitung nach Anspruch 8, **dadurch gekennzeichnet, daß** das weitere Polymer aus der Gruppe ausgewählt ist, die Cellulosen, Cellulosederivate, Polyvinylpyrrolidone, Polyethylenoxide, Pullulan, hydroxypropylierte Tapiokastärke und Alginate umfaßt.

10. Zubereitung nach einem der vorangehenden Ansprüche, **dadurch gekennzeichnet, daß** sie einen oder mehrere Hilfs- oder Zusatzstoffe umfaßt, vorzugweise aus der Gruppe, die Glycerin, Sorbidex, Sucralose, Menthol und Farbstoffe umfaßt.

11. Zubereitung nach Anspruch 1, **dadurch gekennzeichnet, daß** sie eine Zusammensetzung gemäß nachfolgender Tabelle aufweist:
| Komponente | Anteil |
|---|---|
| Polyvinylalkohol (voll-/teilhydrolysiert) | 5-70 Gew.-% |
| Ölige Komponente, wie in Anspruch 1 definiert | 30-80 Gew.-% |
| Hydroxypropylmethylcellulose | 0-35 Gew.-% |
| Natriumcarbocymethylcellulose | 0-35 Gew.-% |
| Glycerin | 0-20 Gew.-% |
| Sorbidex | 0-20 Gew.-% |
| Sucralose | 0- 2 Gew.-% |
| Menthol | 0-10 Gew.-% |
| Bitterfenchelöl | 0- 5 Gew.-% |
| Pfefferminzöl | 0- 5 Gew.-% |
| Kümmelöl | 0- 5 Gew.-% |
| Summe | 100 Gew.-% |

12. Zubereitung nach Anspruch 1, **dadurch gekennzeichnet, daß** sie keine grenzflächenaktiven Substanzen enthält.

13. Verfahren zur Herstellung einer Zubereitung nach einem der vorangehenden Ansprüche, **dadurch gekennzeichnet, daß** eine Unterlage mit einer Masse, umfassend mindestens einen voll- oder teilhydrolysierten Polyvinylalkohol und mindestens 30 Gew.-%, bezogen auf den Trockenanteil der Zubereitung, einer öligen Substanz, wie in Anspruch 1 definiert, in einem wäßrigen Lösungsmittel, beschichtet und die Beschichtung getrocknet wird.

14. Verfahren nach Anspruch 13, **dadurch gekennzeichnet, daß** das Lösungsmittel Wasser ist.

15. Verfahren nach Anspruch 13 oder 14, **dadurch gekennzeichnet, daß** die Herstellung ohne Zusatz von grenzflächenaktiven Substanzen erfolgt.

16. Zubereitung nach einem der Ansprüche 1 bis 12 zur Verwendung als Carminativum zur Behandlung von Beschwerden des Magen-Darm-Traktes.

## Claims

1. Water-soluble, solid, film-shaped preparation for oral administration, comprising at least one film-forming polymer from the group of completely and partially hydrolyzed polyvinyl alcohols and at least one oily substance which is incorporated into the film-forming polymer and whose fraction is at least 30 % by weight, based on the dry fraction of the preparation, where the preparation comprises at least one oily substance selected from the group consisting of mineral oils and synthetic oils, which are esters of dicarboxylic acids, and silicone oils, and also if desired additionally at least one oily substance selected from the group consisting of fatty oils and essential oils.

2. Preparation according to Claim 1 **characterized in that** the partially hydrolyzed polyvinyl alcohol is selected from the group of partially hydrolyzed polyvinyl alcohols which have a degree of hydrolysis of about 88 %.

3. Preparation according to Claim 1 or 2, **characterized in that** the partially hydrolyzed polyvinyl alcohol has a viscosity of from 4 to 40 mPa·s as 4 % strength aqueous solution at 20 °C.

4. Preparation according to Claim 1, **characterized in that** the oily substance is selected from the group which consists of simethicone and dimethicone.

5. Preparation according to one of the preceding claims, **characterized in that** the fraction of the oily substance is at least 40 % by weight, preferably at least 50 % by weight, based on the dry fraction of the preparation.

6. Preparation according to one of the preceding claims, **characterized in that** the fraction of oily substance is at most 80 % by weight, preferably at most 60 % by weight, based on the dry fraction of the preparation.

7. Preparation according to one of the preceding claims, **characterized in that** it comprises at least one oily substance from the group consisting of bitter fennel oil, peppermint oil and caraway oil.

8. Preparation according to one of the preceding claims, **characterized in that** it comprises at least one further hydrophilic polymer.

9. Preparation according to Claim 8, **characterized in that** the further polymer is selected from the group which comprises celluloses, cellulose derivatives, polyvinylpyrrolidones, polyethylene oxides, pullulan, hydroxypropylated tapioca starch and alginates.

10. Preparation according to one of the preceding claims, **characterized in that** it comprises one or more auxiliaries or additives, preferably from the group which comprises glycerol, sorbidex, sucralose, menthol and dyes.

11. Preparation according to Claim 1, **characterized in that** it has a composition according to the table below:
| Component | Fraction |
|---|---|
| Polyvinyl alcohol (completely/partially hydrolyzed) | 5-70 % by weight |
| Oily component, as defined in Claim 1 | 30-80 % by weight |
| Hydroxypropylmethylcellulose | 0-35 % by weight |
| Sodium carboxymethylcellulose | 0-35 % by weight |
| Glycerol | 0-20 % by weight |
| Sorbidex | 0-20 % by weight |
| Sucralose | 0-2 % by weight |
| Menthol | 0-10 % by weight |
| Bitter fennel oil | 0-5 % by weight |
| Peppermint oil | 0-5 % by weight |
| Caraway oil | 0-5 % by weight |
| Total | 100 % by weight |

12. Preparation according to Claim 1, **characterized in that** it comprises no interface-active substances.

13. Process for producing a preparation according to one of the preceding claims, **characterized in that** a substrate is coated with a mass comprising at least one completely or partially hydrolyzed polyvinyl alcohol and at least 30 % by weight, based on the dry fraction of the preparation, of an oily substance, as defined in Claim 1, in an aqueous solvent, and the coating is dried.

14. Process according to Claim 13, **characterized in that** the solvent is water.

15. Process according to Claim 13 or 14, **characterized in that** the preparation takes place without the addition of interface-active substances.

16. Preparation according to one of Claims 1 to 12 for use as carminative for treating complaints of the gastrointestinal tract.

## Revendications

1. Préparation en forme de film, solide, soluble dans l'eau et destinée à l'administration orale, comprenant au moins un polymère filmogène du groupe des alcools polyvinyliques complètement ou partiellement hydrolysés et au moins une substance huileuse, qui est incorporée dans le polymère filmogène et dont la proportion vaut au moins 30 % en poids, rapportée à la proportion à sec de la préparation, dans laquelle la préparation contient au moins une substance huileuse sélectionnée dans le groupe composé d'huiles minérales et d'huiles synthétiques, qui sont des esters d'acides dicarboxyliques, et d'huiles de silicone, ainsi que, au choix en plus, au moins une substance huileuse sélectionnée dans le groupe des huiles grasses et des huiles essentielles.

2. Préparation selon la revendication 1, **caractérisée en ce que** l'alcool polyvinylique partiellement hydrolysé est sélectionné dans le groupe des alcools polyvinyliques partiellement hydrolysés, qui présentent un degré d'hydrolyse d'environ 88 %.

3. Préparation selon la revendication 1 ou 2, **caractérisée en ce que** le polyalcool polyvinylique partiellement hydrolysé présente, comme solution aqueuse à 4 %, une viscosité de 4 à 40 mPa.s à 20°C.

4. Préparation selon la revendication 1, **caractérisée en ce que** la substance huileuse est sélectionnée dans le groupe qui se compose de la siméthicone et du diméthicone.

5. Préparation selon l'une quelconque des revendications précédentes, **caractérisée en ce que** la proportion de la substance huileuse vaut au moins 40 % en poids, de préférence au moins 50 % en poids, rapportée à la proportion à sec de la préparation.

6. Préparation selon l'une quelconque des revendications précédentes, **caractérisée en ce que** la proportion de la substance huileuse vaut au maximum 80 % en poids, de préférence au maximum 60 % en poids, rapportée à la proportion à sec de la préparation.

7. Préparation selon l'une quelconque des revendications précédentes, **caractérisée en ce qu'**elle contient au moins une substance huileuse du groupe composé de l'huile essentielle d'aneth, l'huile essentielle de menthe poivrée et l'huile essentielle de carvi.

8. Préparation selon l'une quelconque des revendications précédentes, **caractérisée en ce qu'**elle comprend au moins un autre polymère hydrophile.

9. Préparation selon la revendication 8, **caractérisée en ce que** l'autre polymère est sélectionné dans le groupe, qui comprend des celluloses, des dérivés de cellulose, la polyvinylpyrrolidone, des oxydes de polyéthylène, le pullulane, l'amidon de tapioca hydroxypropylé et des alginates.

10. Préparation selon l'une quelconque des revendications précédentes, **caractérisée en ce qu'**elle comprend une ou plusieurs substances d'addition, de préférence du groupe qui comprend la glycérine, le sorbidex, le sucralose, le menthol et des colorants.

11. Préparation selon la revendication 1, **caractérisée en ce qu'**elle présente une composition selon le tableau suivant:
| Composant | Proportion |
|---|---|
| Alcool polyvinylique complètement/partiellement hydrolysé) | 5 - 70 % en poids |
| Composant huileux, comme défini dans la revendication 1 | 30 - 80 % en poids |
| Hydroxypropylméthylcellulose | 0 - 35 % en poids |
| Carboxyméthylcellulose de sodium | 0 - 35 % en poids |
| Glycérine | 0 - 20 % en poids |
| Sorbidex | 0 - 20 % en poids |
| Sucralose | 0 - 2 % en poids |
| Menthol | 0 - 10 % en poids |
| Huile essentielle d'aneth | 0 - 5 % en poids |
| Huile essentielle de menthe poivrée | 0 - 5 % en poids |
| Huile essentielle de carvi | 0 - 5 % en poids |
| Total | 100 % en poids |

12. Préparation selon la revendication 11, **caractérisée en ce qu'**elle ne contient pas de substances tensioactives.

13. Procédé de production d'une préparation selon l'une quelconque des revendications précédentes, **caractérisé en ce que** l'on revêt un support, dans un agent solvant aqueux, avec une masse comprenant au moins un alcool polyvinylique complètement ou partiellement hydrolysé et au moins 30 % en poids, rapportés à la proportion à sec de la préparation, d'une substance huileuse, telle qu'elle est définie dans la revendication 1, et on sèche le revêtement.

14. Procédé selon la revendication 13, **caractérisé en ce que** l'agent solvant est l'eau.

15. Procédé selon la revendication 13 ou 14, **caractérisé en ce que** l'on effectue la production sans ajout de substances tensioactives.

16. Préparation selon l'une quelconque des revendications 1 à 12 à utiliser comme carminatif pour le traitement des douleurs du tractus gastro-intestinal.
